# EUROPEAN PATENT SPECIFICATION

(11) **EP 3 446 641 B1**
(45) Date of publication and mention of the grant of the patent: **06.04.2022**
(21) Application number: 18190152.1
(22) Date of filing: 22.08.2018
(51) Int. Cl.: A61B 17/072, A61B 90/00, A61B 17/00

(54) **CONTACTLESS LOADING UNIT DETECTION**
KONTAKTLOSER NACHLADEMAGAZINNACHWEIS
DÉTECTION D'UNITÉ DE CHARGEMENT SANS CONTACT

(30) Priority: 23.08.2017 US 201762549294 P; 24.07.2018 US 201716043230
(43) Date of publication of application: 27.02.2019
(60) Divisional application: 22158936.9
(73) Proprietor: Covidien LP, Mansfield, MA 02048 (US)
(72) Inventor: PANTAZIS, John, Stratford, Connecticut 06614 (US); CALDERONI, Anthony, Bristol, Connecticut 06010 (US)
(74) Representative: Maschio, Antonio

(56) References cited:
- EP-A1- 3 064 146
- EP-A2- 2 923 653
- EP-A2- 2 923 653
- EP-A2- 2 992 839
- JP-A- 2016 011 864
- US-A1- 2011 017 801

## Description

### BACKGROUND

### 1. Technical Field

The present disclosure relates to surgical instruments and, more specifically, to surgical instruments having contactless detection systems for determining connection between components of the surgical instrument.

### 2. Discussion of Related Art

Surgical instruments having a handle and a loading unit releasably coupled to the handle are known. Generally, the loading unit receives mechanical input from the handle to actuate a tool of the loading unit.

Some surgical instruments include a detection system to identify and notify a clinician when the loading unit is properly coupled to the handle. Generally, the detection system includes contacts and a mechanical switch. In such instruments, the contacts may be exposed to bodily fluids, e.g., blood or saline. This fluid exposure may result in malfunctioning of the detection system.

In addition, surgical instruments that include detection systems having exposed contacts may be damaged during resterilazation of the surgical instruments. More specifically, during an autoclave process, exposed electrical contacts may be susceptible to damage from the steam and the high-pressure fluids used in the autoclave process. For example, during the autoclave process the exposed electrical contacts may corrode, form dendtric growths, or electro-plate.

Accordingly, a continuing need exists for detection systems that are not susceptible fluid ingress and/or damage from a resterilization process. EP 3 064 146 A1 and EP 2 923 653 A2 disclose surgical instruments employing a GMR sensor to detect proper attachment of a handle to a loading unit.

JP 2016 011864 A discloses a GMR sensor in the form of an IC.

### SUMMARY

The invention is defined in the appended claims. In an aspect of the present disclosure, a loading unit detection system includes an elongate member, a loading unit, a magnet, and a giant magneto-resistance integrated circuit ("GMR IC"). The elongate member defines a receiver. The loading unit includes a connector that is configured to be received within the receiver to releasably couple the loading unit to the elongate member. The magnet is supported on the connector and is configured to translate relative to the elongate member as the connector is received within the receiver. The GMR IC is embedded within the elongate member and is configured to output a differential voltage in response to a magnetic field produced by the magnet of the loading unit. The differential voltage is indicative of the position of the loading unit within the receiver of the elongate member. The magnet may be embedded within the connector of the loading unit.

In aspects, the loading unit detection system includes a secondary magnet that is embedded within the loading unit such that the differential voltage of the GMR IC induced by a combined magnetic field of the magnet and the secondary magnet is indicative of the position of the loading unit within the receiver of the elongate member and the type of the loading unit.

In some aspects, the loading unit detection system includes an operational amplifier and a latch. The operational amplifier may receive the differential voltage from the GMR IC and transmit an amplified output to the latch. The latch may have a first state when the amplified output is below a threshold and a second state when the amplified output is above the threshold. The amplified output may be below the threshold when the connector is in an uncoupled state with the receiver and above the threshold when the receiver is in a coupled state with the receiver. The operation amplifier and the latch may be integrated with the GMR IC.

In certain aspects, the loading unit detection system includes an instrument amplifier and a filter. The instrument amplifier receives the differential voltage from the GMR IC and outputs an amplified signal to the filter. The filter may be configured to output a filtered signal indicative of the position of the connector relative to the receiver and a type of the loading unit.

In another aspect of the present disclosure, a surgical instrument includes a handle, an adapter, a loading unit, and a loading unit detection system. The adapter is releasably coupled to the handle. The loading unit is releasably coupled to the adapter. The loading unit detection system is configured to detect coupling of the loading unit to the adapter. The loading unit detection system includes a magnet and a GMR IC. The magnet is supported on the loading unit and translates relative to the elongate member as the loading unit is coupled to the adapter. The GMR IC is embedded within the adapter and is configured to output a differential voltage in response to a magnetic field produced by the magnet of the loading unit. The differential voltage produced by the GMR IC is indicative of the position of the loading unit within the elongate member.

According to the invention, the adapter defines a receiver and the loading unit includes a connector. The connector may be received within the receiver to releasably couple the loading unit to the adapter. The magnet may be embedded within the connector of the loading unit. The GMR IC may be embedded within the receiver of the adapter.

In some aspects, the surgical instrument includes a microprocessor that is disposed within the handle. The microprocessor may receive a signal from the GMR IC that is indicative of the position of the loading unit relative to the adapter. The signal received by the microprocessor may be indicative of the type of loading unit.

Further, to the extent consistent, any of the aspects described herein may be used in conjunction with any or all of the other aspects described herein.

In a further aspect of the present disclosure, a surgical loading unit includes a pair of jaws, one of the jaws including a staple cartridge, and the other of the jaws having an anvil for forming staples. The loading unit has a loading unit detection system that includes a device for outputting an electromagnetic field. The loading unit is configured for use in a surgical system that includes a sensor for sensing the electromagnetic field, and communicating information about the loading unit to a microprocessor that is part of the surgical system. The loading unit can include an elongate member, and the detection system can be mounted on the elongate member, or the detection system can be mounted on the jaws. The detection system can be a giant magneto-resistance integrated circuit ("GMR IC"). The surgical system can include an elongate member defining a receiver and the loading unit can include a connector that is configured to be received within the receiver to releasably couple the loading unit to the elongate member. The magnet is supported on the connector. The magnet can be configured to translate relative to the elongate member as the connector is received within the receiver. The GMR IC is configured to output a differential voltage in response to a magnetic field produced by the magnet of the loading unit. The differential voltage can be indicative of the position of the loading unit within the receiver of the elongate member, indicative of characteristics of the loading unit, and can convey other information. The magnet may be embedded within the connector of the loading unit.

### BRIEF DESCRIPTION OF THE DRAWINGS

Various aspects of the present loading unit detection systems for surgical instruments are described hereinbelow with reference to the drawings, which are incorporated in and constitute a part of this specification, wherein:
FIG. 1 is a perspective view of a surgical instrument provided in accordance with the present disclosure including a handle, an adapter coupled to the handle, and a loading unit coupled to the adapter;
FIG. 2 is a perspective view of the surgical instrument of FIG. 1 with the loading unit separated from the adapter;
FIG. 3 is an enlarged view of the indicated area of detail of FIG. 2;
FIG. 4 is a side view of a distal portion of the adapter and a proximal portion of the loading unit of FIG. 3 with parts separated;
FIG. 5 is a side view of the distal portion of the adapter and the proximal portion of the loading unit of FIG. 3 with the loading unit coupled to the adapter;
FIG. 6 is a schematic of a detection circuit provided in accordance with the present disclosure; and
FIG. 7 is a schematic of another detection circuit provided in accordance with the present disclosure.

### DETAILED DESCRIPTION

Embodiments of the present loading unit detection systems for surgical instruments are now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to that portion of the device or component thereof that is closest to the clinician and the term "distal" refers to that portion of the device or component thereof that is farthest from the clinician.

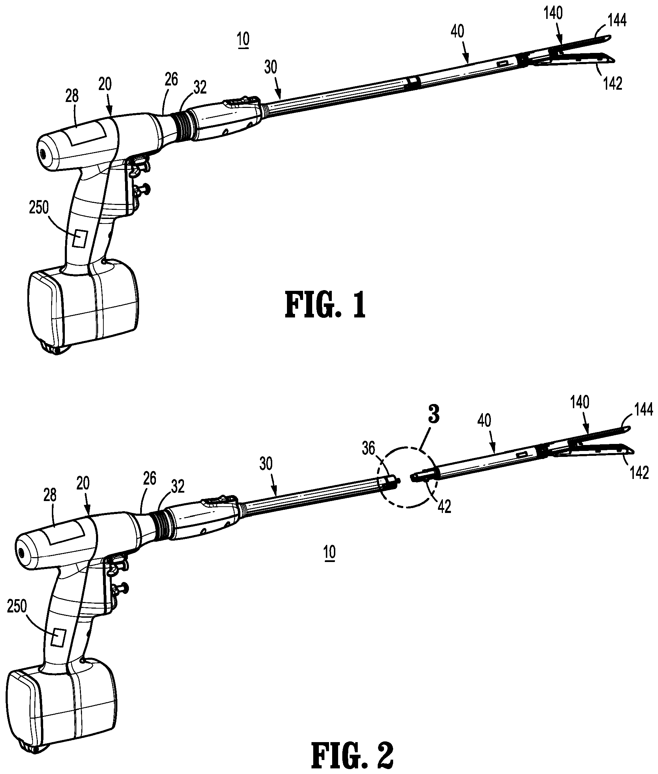

Referring now to FIGS. 1-3, a surgical instrument 10 is provided in accordance with the present disclosure including a handle 20, an adaptor 30, and a loading unit 40. The adaptor 30 includes a proximal portion having a handle connector 32. The handle 20 defines an adaptor receiver 26 for receiving the handle connector 32 to releasably couple the adaptor 30 to the handle 20. The loading unit 40 has a proximal portion includes a loading unit connector 42. The adaptor 30 has a distal portion that defines a loading unit receiver 36 that is positioned to releasably couple the disposable loading unit 40 to the adaptor 30. The loading unit 40 includes an end effector assembly 140 including first and second jaw members 142, 144 that are moveable relative to one another and are configured to act on tissue. It is contemplated that the adapter 30 may be fixed to the handle 20 such that the adapter 30 defines an elongate portion extending from the handle 20.

An exemplary embodiment of a surgical instrument is disclosed in commonly owned U.S. Patent No. 9,055,943.

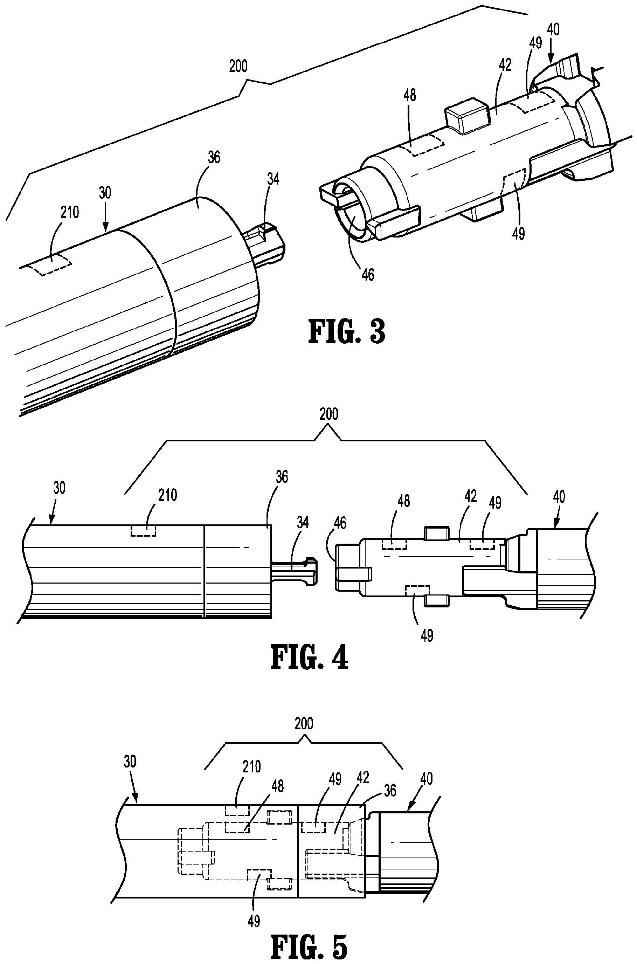

With particular reference to FIG. 3, the adapter 30 includes a translatable shaft 34 that extends distally through the loading unit receiver 36 and is configured to transfer mechanical energy from the adapter 30 to the loading unit 40 to actuate one or more functions of the end effector assembly 140 (FIG. 2), e.g., approximation and/or firing. The loading unit connector 42 of the loading unit 40 defines a shaft receiver 46 that receives the shaft 34 of the adapter 30. When the loading unit connector 42 is fully received within the loading unit receiver 36 the shaft 34 is coupled to the shaft receiver 46 such that translation of the shaft 34 actuates the end effector assembly 140.

With reference to FIGS. 3-5, the adapter 30 includes a contactless loading unit detection system 200 to determine the position of the loading unit connector 42 within the loading unit receiver 36. The loading unit detection system 200 includes a giant magneto-resistance integrated circuit (GMR IC) 210 embedded within a distal portion of the adapter 30 and the loading unit 40 includes a magnet 48 embedded within the loading unit connector 42. As detailed below, the GMR IC 210 is configured to output a differential voltage based upon the magnetic field induced in the GMR IC 210 by the magnet 48 of the loading unit 40. It will be appreciated that the differential voltage of the GMR IC 210 increases as a distance between the magnet 48 and the GMR IC 210 decreases.

In embodiments, the GMR IC 210 may be embedded within or adjacent the loading unit receiver 36 of the adapter 30 and/or the magnet 48 may be embedded within or adjacent the loading unit connector 42. By embedding both the GMR IC 210 and the magnet 48 within the loading unit receiver 36 and the loading unit connector 42, respectively, the loading unit detection system 200 does not include any exposed contacts. As such, fluid ingress into the loading unit detection system 200 is prevented. By preventing fluid ingress into the loading unit detection system 200, false indemnification of proper coupling of the loading unit 40 with the adapter 30 may be minimized.

The induced differential voltage of the GMR IC 210 is indicative of the position of the magnet 48 relative to the GMR IC 210 and thus, indicative of the position of the loading unit connector 42 of the loading unit 400 within the loading unit receiver 36 of the adapter 30. When the differential voltage of the GMR IC 210 reaches a predetermined threshold value, the loading unit connecter 42 is fully received within the loading unit receiver 36 (FIG. 5) such that the loading unit 40 is coupled to the adapter 30.

The GMR IC 210 may provide an analog or digital output indicative of the position of the loading unit connector 42 within the loading unit receiver 36. The handle 20 may include an indicator 28 configured to provide a visual indication of the position of the loading unit 40 within the adapter 30 and/or provide a visual indication when the loading unit 40 is coupled to the adapter 30.

In embodiments, the differential voltage of the GMR IC 210 may be used to identify the type of loading unit that is coupled to the adapter 30. For example, the loading unit 40 may include one or more additional magnets 49 that are/is configured to generate a unique magnetic field in the GMR IC 210 when the loading unit 40 is coupled to the adapter 30 such that the differential voltage of the GMR IC 210 is characteristic of the loading unit 40 that is coupled to the adapter 30. For example, in a linear stapling loading unit, the length of the staple line, staple size or sizes, characteristics such as whether the unit has a knife, dissection tip, buttress, etc., can be indicated. An indication that the unit has been used, is unused, or is within or beyond a certain prescribed number of uses, is contemplated.

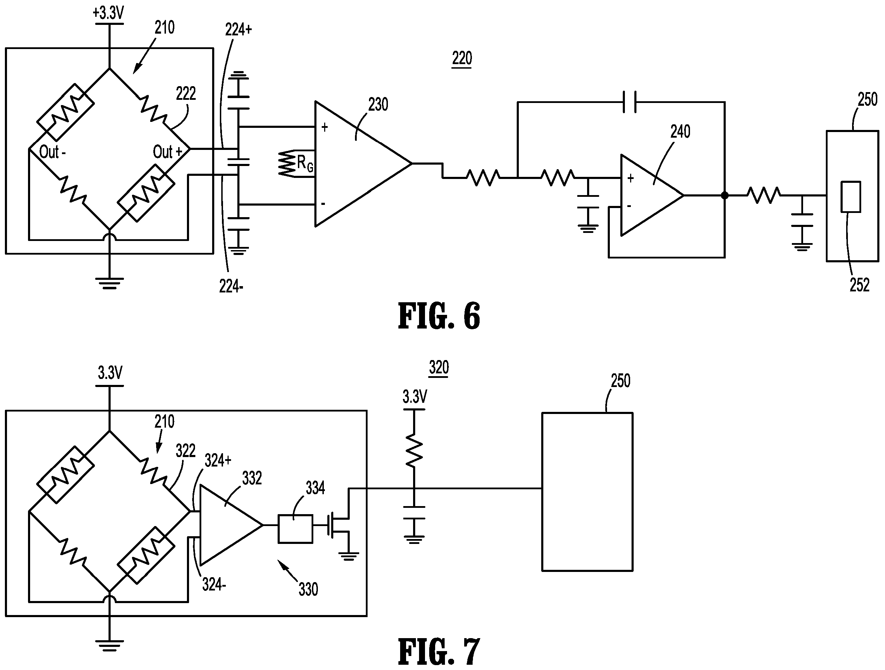

The differential voltage of the GMR IC 210 is received within a GMR circuit, e.g., GMR circuit 220 (FIG. 6) or GMR circuit 320 (FIG. 7) detailed below, and outputted to a microprocessor 250 (FIG. 1) disposed within the handle 20 of the surgical instrument 10. The microprocessor 250 analyzes the output of the GMR circuit to detect and/or identify a loading unit 40 is properly coupled to the adapter 30.

With reference to FIG. 6, a schematic of an illustrative GMR circuit 220 capable of detecting and identifying the presence and/or relative position of a loading unit, e.g., loading unit 40, is shown. The GMR circuit 220 includes the GMR IC 210 in the form of a partially shielded bridge 222 with positive and negative outputs 224+, 224-. The GMR IC 210 outputs a differential voltage signal based on a magnetic field induced in the GMR IC 210 by the magnets 48, 49 (FIG. 3). Specifically, as the magnetic field experienced by the GMR IC 210 varies, e.g., as the position of the magnets 48, 49 relative to the GMR IC 210 changes, the differential voltage of the output 224 varies. The outputs 224 of the GMR IC 210 are electrically coupled to an instrumentation amplifier 230 which outputs an amplified signal. The amplified signal may pass through a filter, e.g., low pass filter 240 before being transmitted to the microprocessor 250. The filtered signal of the low pass filter 240 may be transmitted to the analog to digital converter (ADC) 252 of the microprocessor 250 which analyzes the signal to identify the loading unit 40 based on a unique voltage signature and to determine when the loading unit 40 is coupled to the adapter 30. The microprocessor 250 may provide visual and/or audible indicia of the type of loading unit 40 and/or when the loading unit 40 is coupled to the adapter 30. The visual indicia may be provided on the display 28 (FIG. 1).

With reference to FIG. 7, a schematic of another illustrative GMR circuit 320 capable of detecting presence and/or relative position of a loading unit, e.g., loading unit 40, is shown. The GMR circuit 320 includes the GMR IC 210 in the form of a partially shielded bridge 322 with positive and negative outputs 324+, 324-. The GMR IC 210 outputs a differential voltage signal base on a magnetic field induced in the GMR IC 210 by the magnet 48. Specifically, as the magnetic field experienced by the GMR IC 210 varies, e.g., as the position of the magnet 48 relative to the GMR IC 210 changes, the differential voltage of the output 324 varies. The outputs 324 of the GMR IC 210 are electrically coupled to a comparator circuit 330 configured to detect when a signal from the GMR IC 210 exceeds a threshold value indicative of the position of the magnet 48 when the loading unit 40 is coupled to the adapter 30. The comparator circuit 330 may include an operational amplifier 332 and a latch 334 such that a connection signal is outputted from the latch 334 to the microprocessor 250 when the voltage of the GMR IC 210 is above the threshold value. It will be appreciated that no connection signal is outputted from the latch 334 when the voltage of the GMR IC 210 is below the threshold value. Alternatively, the latch 334 can be reversed such that the connection signal is outputted when the voltage of the GMR IC 210 is below the threshold value and is not outputted when the voltage of the GMR IC 210 is above the threshold value. The comparator circuit 330 may be integrated with the shielded bridge 322 to form an integrated GMR IC 210. The microprocessor may provide visual and/or audible indicia when the loading unit 40 is coupled to the adapter 30. The visual indicia may be provided on the display 28 (FIG. 1).

In some embodiments, the adapter 30 includes a translating element (not shown) which is translated proximally as the loading unit connector 42 is received within the loading unit receiver 36. In such embodiments, the magnet 48 and/or magnet 49 may be embedded within the translating element and the GMR IC 210 may be embedded within the adapter 30 at a position to determine the position of the magnet 48. In these embodiments, the entire detection system 200 may be disposed within the adapter 30 and/or the GMR IC 210 may be positioned in a central or proximal portion of the adapter 30 remote from the loading unit receiver 36. In some embodiments, the magnet 48 is embedded in the translating element and the magnets 49 are embedded within the loading unit connector 42 to induce a unique voltage in the GMR IC 210. For an exemplary translating element reference may be made to the detection link ring, the switch link ring, the switch link, the switch pin, or the switch button of the loading unit detection assembly of U.S. Patent Publication No. 2013/0324979, the entire contents of which are hereby incorporated by reference.

It will be appreciated that the handle 20 and the adapter 30 may also include a contactless detection system similar to the contactless detection system 200 detailed above to detect when and/or the type of adapter 30 coupled to the handle 20.

In any of the embodiments disclosed herein, the microprocessor 250 can be provided in a hand-held housing, a remote console, or some other device. The loading unit can be configured to be used in a robotic surgical system. Also, the contactless detection system can include technologies other than magnetic, such as induction.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and forms part of the invention as long as it is within the scope of the appended claims. Therefore, the above description should not be construed as limiting, but merely as exemplifications of particular embodiments. Those skilled in the art will envision other modifications within the scope of the claims appended hereto.

## Claims

1. A loading unit detection system (200) comprising:
an adaptor (30) configured to releasably couple to a handle (20) of a surgical instrument.
wherein the adaptor defines an elongate member defining a receiver (36);
a loading unit (40) including a connector (42) configured to be received within the receiver to releasably couple the loading unit to the elongate member;
a magnet (48) supported on the connector, the magnet configured to translate relative to elongate member as the connector is received within the receiver; and
a GMR IC (210) embedded within the elongate member, the GMR IC configured to output a differential voltage in response to a magnetic field produced by the magnet of the loading unit, the differential voltage being indicative of the position of the loading unit within the receiver of the elongate member.

2. The system (200) according to claim 1, wherein the magnet (48) is embedded within the connector (42) of the loading unit (40).

3. The system (200) according to claim 1 or claim 2, further comprising a secondary magnet (49) embedded within the loading unit (40) such that the differential voltage of the GMR IC (210) induced by a combined magnetic field of the magnet (48) and the secondary magnet is indicative of the position of the loading unit within the receiver (36) of the elongate member and the type of the loading unit.

4. The system (200) according to any preceding claim, further comprising an operational amplifier (332) and a latch (334), the operational amplifier receiving the differential voltage from the GMR IC (210) and transmitting amplified output to the latch, the latch having a first state when the amplified output is below a threshold and a second state when the amplified output is above the threshold.

5. The system (200) according to claim 4, wherein the amplified output is below the threshold when the connector (42) is in an uncoupled state with the receiver (36) and above the threshold when the receiver is in the coupled state with the receiver.

6. The system (200) according to claim 4 or claim 5, wherein the operation amplifier (332) and latch (334) are integrated with the GMR IC (210).

7. The system (200) according to any preceding claim, further comprising an instrument amplifier (230) and a filter (240), the instrument amplifier receiving the differential voltage from the GMR IC (210) and then outputting an amplified signal to the filter, the filter configured to output a filtered signal indicative of the position of the connector (42) relative to the receiver (36) and a type of the loading unit (40).

8. A surgical instrument (10) comprising:
a handle (20);
the adapter (30) releasably coupled to the handle;
the loading unit detection system (200) of any preceding claim.

9. The surgical instrument (10) according to claim 8, wherein the GMR IC (210) is embedded within the receiver (36) of the adapter (30).

10. The surgical instrument (10) according to any of claims 8 to 9, further comprising a microprocessor (250) disposed within the handle (20), the microprocessor receiving a signal from the GMR IC (210) indicative of the position of the loading unit (40) relative to the adapter (30).

11. The surgical instrument (10) according to claim 10, wherein the signal received by the microprocessor (250) is indicative of the type of loading unit (40).

## Patentansprüche

1. Ladeeinheitserfassungssystem (200), umfassend:
einen Adapter (30), der konfiguriert ist, um mit einem Griff (20) eines chirurgischen Instruments lösbar gekoppelt zu werden,
wobei der Adapter ein längliches Element definiert, das einen Empfänger (36) definiert;
eine Ladeeinheit (40), die einen Verbinder (42) beinhaltet, der konfiguriert ist, um innerhalb des Empfängers empfangen zu werden, um die Ladeeinheit mit dem länglichen Element lösbar zu koppeln;
einen Magneten (48), der auf dem Verbinder gelagert ist, wobei der Magnet konfiguriert ist, um relativ zu dem länglichen Element verschoben zu werden, während der Verbinder innerhalb des Empfängers empfangen wird; und
eine GMR-IC (210), die innerhalb des länglichen Elements eingebettet ist, wobei die GMR-IC konfiguriert ist, um eine Differenzspannung als Reaktion auf ein Magnetfeld auszugeben, das durch den Magneten der Ladeeinheit erzeugt wird, wobei die Differenzspannung die Position der Ladeeinheit innerhalb des Empfängers des länglichen Elements anzeigt.

2. System (200) nach Anspruch 1, wobei der Magnet (48) innerhalb des Verbinders (42) der Ladeeinheit (40) eingebettet ist.

3. System (200) nach Anspruch 1 oder 2, ferner umfassend einen sekundären Magneten (49), der innerhalb der Ladeeinheit (40) derart eingebettet ist, dass die Differenzspannung der GMR-IC (210), die durch ein kombiniertes Magnetfeld des Magneten (48) und des sekundären Magneten induziert wird, die Position der Ladeeinheit innerhalb des Empfängers (36) des länglichen Elements und die Art der Ladeeinheit anzeigt.

4. System (200) nach einem der vorhergehenden Ansprüche, ferner umfassend einen Operationsverstärker (332) und ein Latch (334), wobei der Operationsverstärker die Differenzspannung von der GMR-IC (210) empfängt und eine verstärkte Ausgabe an das Latch überträgt, wobei das Latch einen ersten Zustand, wenn die verstärkte Ausgabe unter einem Schwellenwert liegt, und einem zweiten Zustand aufweist, wenn die verstärkte Ausgabe über dem Schwellenwert liegt.

5. System (200) nach Anspruch 4, wobei die verstärkte Ausgabe unter dem Schwellenwert, wenn sich der Verbinder (42) in einem nicht gekoppelten Zustand mit dem Empfänger (36) befindet, und über dem Schwellenwert liegt, wenn sich der Empfänger in dem gekoppelten Zustand mit dem Empfänger befindet.

6. System (200) nach Anspruch 4 oder 5, wobei der Operationsverstärker (332) und das Latch (334) mit der GMR-IC (210) integriert sind.

7. System (200) nach einem der vorhergehenden Ansprüche, ferner umfassend einen Instrumentenverstärker (230) und ein Filter (240), wobei der Instrumentenverstärker die Differenzspannung von der GMR-IC (210) empfängt und dann ein verstärktes Signal an das Filter ausgibt, wobei das Filter konfiguriert ist, um ein gefiltertes Signal auszugeben, das die Position des Verbinders (42) relativ zu dem Empfänger (36) und eine Art der Ladeeinheit (40) anzeigt.

8. Chirurgisches Instrument (10), umfassend:
einen Griff (20);
den Adapter (30), der mit dem Griff lösbar gekoppelt ist;
das Ladeeinheitserfassungssystem (200) nach einem der vorhergehenden Ansprüche.

9. Chirurgisches Instrument (10) nach Anspruch 8, wobei die GMR-IC (210) innerhalb des Empfängers (36) des Adapters (30) eingebettet ist.

10. Chirurgisches Instrument (10) nach einem der Ansprüche 8 bis 9, ferner umfassend einen Mikroprozessor (250), der innerhalb des Griffs (20) angeordnet ist, wobei der Mikroprozessor ein Signal von der GMR-IC (210) empfängt, das die Position der Ladeeinheit (40) relativ zu dem Adapter (30) anzeigt.

11. Chirurgisches Instrument (10) nach Anspruch 10, wobei das Signal, das durch den Mikroprozessor (250) empfangen wird, die Art der Ladeeinheit (40) anzeigt.

## Revendications

1. Système de détection d'unité de chargement (200) comprenant :
un adaptateur (30) conçu pour s'accoupler de manière amovible à une poignée (20) d'un instrument chirurgical,
dans lequel l'adaptateur définit un élément allongé définissant un récepteur (36) ;
une unité de chargement (40) comportant un raccord (42) conçu pour être reçu à l'intérieur du récepteur pour accoupler de manière amovible l'unité de chargement à l'élément allongé ;
un aimant (48) supporté sur le raccord, l'aimant étant conçu pour translater par rapport à l'élément allongé lorsque le raccord est reçu à l'intérieur du récepteur ; et
un CI GMR (210) intégré à l'intérieur de l'élément allongé, le CI GMR étant configuré pour émettre une tension différentielle en réponse à un champ magnétique produit par l'aimant de l'unité de chargement, la tension différentielle indiquant la position de l'unité de chargement à l'intérieur du récepteur de l'élément allongé.

2. Système (200) selon la revendication 1, dans lequel l'aimant (48) est intégré à l'intérieur du raccord (42) de l'unité de chargement (40).

3. Système (200) selon la revendication 1 ou la revendication 2, comprenant en outre un aimant secondaire (49) intégré à l'intérieur de l'unité de chargement (40) de telle sorte que la tension différentielle du CI GMR (210) est induite par un champ magnétique combiné de l'aimant (48) et que l'aimant secondaire indique la position de l'unité de chargement à l'intérieur du récepteur (36) de l'élément allongé et le type d'unité de chargement.

4. Système (200) selon l'une quelconque des revendications précédentes, comprenant en outre un amplificateur opérationnel (332) et un verrou (334), l'amplificateur opérationnel recevant la tension différentielle du CI GMR (210) et transmettant une sortie amplifiée au verrou, le verrou ayant un premier état lorsque la sortie amplifiée est inférieure à un seuil et un second état lorsque la sortie amplifiée est supérieure au seuil.

5. Système (200) selon la revendication 4, dans lequel la sortie amplifiée est inférieure au seuil lorsque le raccord (42) est dans un état désaccouplé avec le récepteur (36) et au-dessus du seuil lorsque le récepteur est dans l'état accouplé avec le récepteur.

6. Système (200) selon la revendication 4 ou la revendication 5, dans lequel l'amplificateur opérationnel (332) et le verrou (334) sont intégrés au CI GMR (210).

7. Système (200) selon l'une quelconque des revendications précédentes, comprenant en outre un amplificateur d'instrument (230) et un filtre (240), l'amplificateur d'instrument recevant la tension différentielle du CI GMR (210) puis émettant un signal amplifié vers le filtre, le filtre étant configuré pour émettre un signal filtré indiquant la position du raccord (42) par rapport au récepteur (36) et un type d'unité de chargement (40).

8. Instrument chirurgical (10) comprenant :
une poignée (20) ;
l'adaptateur (30) accouplé de manière amovible à la poignée ;
le système de détection d'unité de chargement (200) selon l'une quelconque des revendications précédentes.

9. Instrument chirurgical (10) selon la revendication 8, dans lequel le CI GMR (210) est intégré à l'intérieur du récepteur (36) de l'adaptateur (30).

10. Instrument chirurgical (10) selon l'une quelconque des revendications 8 à 9, comprenant en outre un microprocesseur (250) disposé à l'intérieur de la poignée (20), le microprocesseur recevant un signal du CI GMR (210) indiquant la position de l'unité de chargement (40) par rapport à l'adaptateur (30).

11. Instrument chirurgical (10) selon la revendication 10, dans lequel le signal reçu par le microprocesseur (250) indique le type d'unité de chargement (40).
